# EUROPÄISCHE PATENTANMELDUNG

(11) **EP 1 136 063 A2**
(43) Veröffentlichungstag der Anmeldung: **26.09.2001**
(21) Anmeldenummer: 01104420.3
(22) Anmeldetag: 26.02.2001
(51) Int. Cl.: A61K 7/48

(54) **Abrasivum auf Basis von biologischem Material und Verfahren zu dessen Herstellung**

(30) Priorität: 25.02.2000 DE 10008816
(71) Anmelder: Physioderm GmbH & Co. KG, 67065 Ludwigshafen (DE)
(72) Erfinder: Franzen, Martin, 67547 Worms (DE); Bouillon, Günther, Dr., 67434 Neustadt (DE); Frisch, Albert, 67483 Edesheim (DE); Giesen, Rainer, 47809 Krefeld (DE)
(74) Vertreter: Müller-Boré & Partner Patentanwälte

(57) **Zusammenfassung**

Die Erfindung betrifft ein Abrasivum auf Basis von biologischem Material sowie ein Verfahren zu dessen Herstellung. Die Erfindung betrifft ferner die Verwendung des erfindungsgemäßen Abrasivums.

## Beschreibung

Die Erfindung betrifft ein Abrasivum auf Basis von biologischem Material sowie ein Verfahren zu dessen Herstellung. Die Erfindung betrifft ferner die Verwendung des erfindungsgemäßen Abrasivums.

Der Einsatz von körnigem Material in Kosmetika, zum einen in Peelingprodukten zur Entfernung der oberen Hautschichten (Epidermis), zum anderen in Hand- und Hautreinigern zur mechanischen Unterstützung der Reinigungswirkung von Tensiden und auch als Schmutzträger für den abgelösten Schmutz ist seit langem bekannt. Im Stand der Technik wurden als körniges Material zunächst anorganische Materialien, wie Sand, Calzit, Kreide, Marmor, Dolomit, Feldspat und Quarz, oder auch Holzmehl verwendet.

Die anorganischen Materialien weisen jedoch eine starke Schleifwirkung auf und sind daher für viele Anwendungen nicht geeignet.

Natürliches Holzmehl weist einen hohen Keimgehalt und eine dunkle Farbe auf. Es ist daher eine starke Konservierung nötig und es müssen aufhellende Pigmente, wie beispielsweise Titandioxid, zugesetzt werden, um helle, sauber wirkende und daher kosmetisch akzeptable Produkte zu erhalten.

Es wurde auch versucht, Holzmehl durch Kunststoffmehle zu ersetzen, beispielsweise auf Basis von Polyethylen- oder Polyurethanpulvern. Derartige Kunststoffmehle sind jedoch biologisch nicht abbaubar und durch Mahlen nur schwierig in einer gleichmäßigen Kornverteilung zu erhalten.

Ferner wurden als natürliche Abrasivstoffe auch gemahlene Schalen und Kerne von Früchten und Nüssen vorgeschlagen. Gemäß DE 40 38 076 C2 werden derartige Materialien aus natürlichen Schalen und/oder Kernen mit Wasserstoffperoxid im alkalischen Medium in Suspension behandelt, wodurch ein gebleichtes natürliches Schalen- und/oder Kernmehl mit einem geringen Keimgehalt erhalten wird. Dieses Verfahren weist jedoch den erheblichen Nachteil auf, daß das Bleichen des Kern- und Schalenmehls in wasserhaltiger Suspension im alkalischen Milieu durchgeführt wird. Durch diesen Verfahrensschritt fallen große Mengen an Ablaugen an. Im Beispiel 1 der DE 40 38 076 C2 wird dabei ein Bleichverfahren beschrieben, worin der Wassergehalt der das biologische Material und das Bleichmittel umfassenden Mischung weit über 60 Gew.-% beträgt. Die anfallenden großen Mengen an Ablaugen weisen sowohl einen hohen CSB ("Chemischer Sauerstoffbedarf") als auch einen hohen BSB ("Biologischer oder Biochemischer Sauerstoffbedarf") auf. Dies wird durch die Wirkung des alkalischen Prozesses verstärkt, durch welchen die Pflanzenfasern angegriffen bzw. teilweise aufgeschlossen werden und umweltbelastende Stoffe freisetzen. Das Aufschließen der Pflanzenfasern führt außerdem dazu, daß die Ausbeute an gebleichtem Kern- und Schalenmehl verringert wird. Ferner weisen die gebleichten Pflanzenfasern nach dem Abfiltrieren von der Lauge noch einen hohen Wassergehalt von etwa 50% auf und müssen getrocknet werden. Bei diesem Trocknungsschritt besteht jedoch die Gefahr, daß das durch die Bleichbehandlung entkeimte Produkt wieder mit Keimen kontaminiert wird.

Der vorliegenden Erfindung liegt somit die Aufgabe zugrunde, ein Verfahren zur Herstellung eines Abrasivums auf Basis von biologischem Material bereitzustellen, mit welchem ein in der Kosmetik verwendbares Abrasivum mit geringem Keimgehalt und heller Eigenfarbe erhalten werden kann, wobei gleichzeitig aber keine großen Mengen an Ablaugen anfallen sollen. Das daraus resultierende Abrasivum soll lagerfähig und mikrobiologisch akzeptabel sein. Des weiteren soll das Abrasivum gut hautverträglich sein, d.h. es soll einen mit der Haut verträglichen pH-Wert aufweisen.

Diese Aufgabe wird durch die in den Patentansprüchen gekennzeichneten Gegenstände gelöst. Insbesondere betrifft die vorliegende Erfindung ein Verfahren zur Herstellung eines Abrasivums für kosmetische Präparate, umfassend das Mahlen eines biologischen Materials und das Mischen des mindestens einen gemahlenen biologischen Materials mit einem Bleichmittel, wobei die das biologische Material und das Bleichmittel enthaltende Mischung höchstens 60 Gew.-% Wasser enthält und das unmittelbar nach der Behandlung mit dem Bleichmittel erhaltene biologische Material einen pH-Wert von höchstens 5,0, vorzugsweise etwa 3,2 bis 4,4, mehr bevorzugt 3,2 bis 4,0, bestimmt als 10%-ige Suspension in Wasser, aufweist.

Die Lösung der Aufgabe beruht auf der überraschenden Erkenntnis, daß durch die "trockene" Behandlung von gemahlenem biologischem Material mitAktivsauerstoff bei einem pH-Wert von höchstens 5,0, vorzugsweise in einem pH-Bereich von etwa 3,2 bis 4,4, mehr bevorzugt 3,2 bis 4,0, ein Abrasivum hergestellt werden kann, welches den Erfordernissen für einen kosmetischen Einsatz in vorteilhafter Weise genügt. Unter einer "trockenen" Behandlung wird gemäß dieser Erfindung verstanden, daß das Bleichen bzw. Keimfreimachen nicht in einer wäßrigen Suspension, sondern bestenfalls in einer feuchten Masse, durchgeführt wird. Insbesondere sollte die Mischung aus biologischem Material und Bleichmittel höchstens 60 Gew.-% Wasser enthalten.

Erfindungsgemäß können als biologisches Material insbesondere pflanzliche Materialien verwendet werden, wie beispielsweise Gräser, wie Bambus, Heu, Stroh oder Schilf, Teile vom Stamm oder von Ästen diverser Gehölze, wie Kokospalmen und Avocados, Spelzen von Getreide, wie Dinkel, Weizen, Hafer, Roggen und Gerste oder aber auch Luffa Gurken und Rattan. In einer bevorzugten Ausführungsform der vorliegenden Erfindung wird als biologisches Material Kokosmark eingesetzt. Im Rahmen des erfindungsgemäßen Verfahrens können aber auch Schalen von Früchten, wie Walnuß, Kokosnuß und Haselnuß, Kerne von Früchten, wie Pfirsich, Aprikose, Kirsche, Pflaume und Mandel bzw. von Datteln oder Oliven, vorgesehen werden. Die vorstehenden Materialien können einzeln oder als Gemisch von einem oder mehreren davon verwendet werden. Erfindungsgemäß wird das gemahlene biologische Material nicht in Suspension, sondern im "trockenen" Zustand, d.h. so wie das biologische Material anfällt, behandelt. Gemahlenes biologisches Material weist vor der erfindungsgemäßen Behandlung üblicherweise einen Wassergehalt von höchstens 50 Gew.-%, vorzugsweise höchstens 35 Gew.-% auf. Das biologische Material kann vor der Bleichbehandlung bzw. dem Keimfreimachen getrocknet werden. Da nach dem Bleichen in der Regel kein Trocknungsschritt mehr erfolgt und nur geringe Mengen Feuchtigkeit für den Bleichvorgang zugegeben werden, welche nicht wieder entfernt werden müssen, kann so der Wassergehalt des Endprodukts beeinflußt werden, d.h. ein geringerer Wassergehalt des gemahlenen biologischen Materials als Ausgangsprodukt führt bei gleicher zugesetzter Menge an Bleichmittel zu einem geringeren Wassergehalt des gebleichten bzw. keimfrei gemachten Abrasivums. Der Wassergehalt des Endprodukts liegt bei höchstens 60 Gew.-%, vorzugsweise bei höchstens 45 Gew.-% und besonders bevorzugt bei höchstens 20 Gew.-%.

Das biologische Material wird zunächst durch geeignete Technologien zerkleinert und in einer Körnung von 10 bis 2000 *µ*m, vorzugsweise 15 bis 200 *µ*m klassiert. Zum Mahlen können übliche Mühlen eingesetzt werden, beispielsweise Kugelmühlen, Schwingkugelmühlen, Umlaufmühlen, Hammermühlen oder Feinprallmühlen. Zum Klassieren des gemahlenen biologischen Materials ("Mehl") finden ebenfalls herkömmliche Verfahren, wie Sieben, Windsichten und Stromklassieren, Anwendung.

Erfindungsgemäß wird das gemahlene biologische Material anschließend einer "trockenen" Behandlung mit einem Bleichmittel unterworfen. Das Bleichmittel enthält eine Verbindung mit aktivem Sauerstoff, wie Peroxy-Verbindungen. Vorzugsweise enthält das Bleichmittel ein oder mehrere Persäuren. Mehr bevorzugt ist der Einsatz von organischen Peroxysäuren wie Peroxyameisensäure oder Peroxyessigsäure. Im Rahmen des erfindungsgemäßen Verfahrens kann aber auch Wasserstoffperoxid, entweder für sich genommen oder in Kombination mit einer oder mehreren Persäuren, als Bleichmittel eingesetzt werden. Die vorgenannten organischen Peroxysäuren können auch *in situ* hergestellt werden. Gemäß einer derartigen Ausführungsform der vorliegenden Erfindung werden dazu Wasserstoffperoxid, gegebenenfalls Wasser, mindestens eine organische Säure und mindestens eine Mineralsäure, wie Schwefelsäure, vor dem Aufsprühen auf das gemahlene biologische Material gemischt. Der Wasserstoffperoxidgehalt beträgt dabei vorzugsweise 15 bis 40 Gew.-%, der Wassergehalt 5 bis 80 Gew.%, vorzugsweise 5 bis 40 Gew.-%, der Gehalt an organischer Carbonsäure vorzugsweise 10 bis 40 Gew.-% und der Gehalt an Mineralsäure vorzugsweise 0 bis 5 Gew.-%, jeweils bezogen auf das Gesamtgewicht der vorstehenden Mischung.

Das Vermischen der Komponenten des Bleichmittels kann unmittelbar vor dem Aufsprühen mittels eines Mischers in den Zuleitungen des Mischers stattfinden oder alternativ dazu in einem größeren Vorratstank, um dann aufgesprüht zu werden.

Das Bleichmittel kann ferner mindestens einen Moderator in einem Anteil von bis zu 5 Gew.-%, vorzugsweise bis zu 2 Gew.-%, bezogen auf die das biologische Material und das Bleichmittel enthaltende Mischung, enthalten. Als Moderatoren können alkalische Mittel, wie beispielsweise Wasserglas, Soda, Pottasche oder Laugen, verwendet werden.

Das Bleichmittel wird vorzugsweise auf das biologische Material aufgesprüht und mit diesem vermischt und dieses gegebenenfalls mit weiteren Moderatoren und Zuschlagsstoffen behandelt. Vorzugsweise beträgt der Gehalt des biologischen Materials mindestens 60 Gew.-%, vorzugsweise 80 Gew.-%, mehr bevorzugt 90 Gew.-% und besonders bevorzugt 95 Gew.-%, bezogen auf die das biologische Material und das Bleichmittel umfassende Mischung.

Das biologische Material und das Bleichmittel können mit üblichen Mischern kontinuierlich oder chargenweise gemischt werden. Üblicherweise ist der Mischvorgang nach spätestens 30 min abgeschlossen. Vorzugsweise enthält die das biologische Material und das Bleichmittel umfassende Mischung höchstens 60 Gew.-%, vorzugsweise höchstens 40 Gew.-% Wasser und besonders bevorzugt höchstens 15 Gew.-% Wasser.

Nach dem Mischen kann eine bis zu 24stündige Nachreifung stattfinden, während der sich das Material bis auf etwa 60 bis 70°C erwärmen kann. Diese Art der Nachreifung ist um so ausgeprägter, je holzähnlicher das biologische Material ist. Während dieser Nachreifung wird der Veredelungsprozeß vollendet. Die Nachreifung kann durch Erwärmen der Mischung auf etwa 40 bis 80°C beschleunigt werden.

Das Peroxid zerfällt vollständig und nach etwa 10 Tagen sind im fertigen Produkt keine Peroxide mehr nachweisbar. Als Zerfallsprodukte aus dem Bleichmittel können neben Sauerstoff und Wasser organische Carbonsäuren entstehen. Beispielsweise sind bei einer Menge von 15 Gew.-% Carbonsäure im Bleichmittel und einem Einsatz von 5 Gew.-% Bleichmittel, bezogen auf das Gesamtgewicht von Abrasivum und Bleichmittel, maximal etwa 0,75 Gew.-% Carbonsäure im fertigen Produkt enthalten. Dieser geringe Anteil an Carbonsäure im erfindungsgemäßen Abrasivum begünstigt die Bildung von Salzen, welche als schwach saurer Puffer den Einsatz von milden bzw. gut verträglichen Konservierungsstoffen, wie beispielsweise Benzoesäure und deren Derivate, in den kosmetischen Endprodukten erlauben. Gegebenenfalls können diese Carbonsäuren aber auch aus dem Produkt entfernt werden.

Gemäß einer Ausführungsform kann das behandelte biologische Material nach dem Nachreifen gewaschen werden. Vorzugsweise entfällt jedoch dieser Schritt des Nachwaschens.

Figur 1 ist eine schematische Darstellung gemäß einer Ausführungsform (kontinuierliches Verfahren) des Mischungsschritts des erfindungsgemäßen Verfahrens.

Figur 2 ist eine schematische Darstellung gemäß einer weiteren Ausführungsform (chargen-weise) des Mischungsschritts des erfindungsgemäßen Verfahrens.

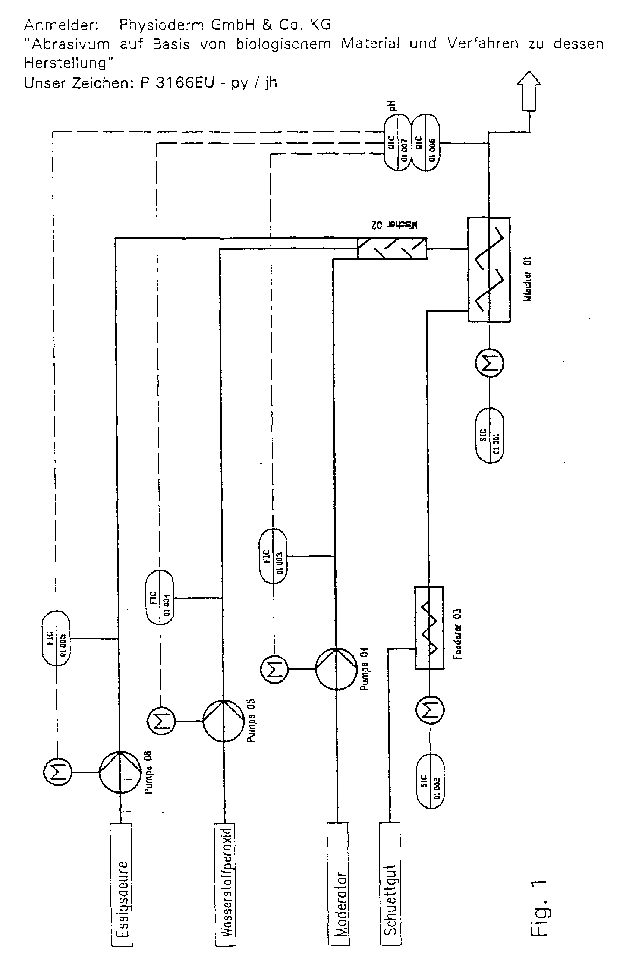

Gemäß einer Ausführungsform kann das erfindungsgemäße Abrasivum in einem kontinuierlichen Verfahren hergestellt werden. In Figur 1 ist ein derartiger kontinuierlicher Mischvorgang gezeigt. Das Rohmaterial, d.h. das gemahlene biologische Material, wird als Schüttgut mit einem Dosierförderer (03) dem Mischer (01) kontinuierlich zugeführt. Die flüssigen Inhaltsstoffe, d.h. das Bleichmittel und gegebenenfalls ein Moderator und/oder eine Mineralsäure, werden proportional zur Förderleistung des Schüttguts und gemäß der jeweiligen Herstellvorschrift mit geeigneten Pumpen (04) bis (06), beispielsweise Zahnradpumpen, gleichfalls dem Mischer zugeführt. In einer vorgeschalteten Mischstufe (02), beispielsweise einem Statikmischer, werden die Komponenten des Bleichmittels homogenisiert. Das so hergestellte Bleichmittel wird im Mischer (01) auf das mechanisch fluidisierte Schüttgut aufgesprüht. Das Reaktionsgemisch, welches das Bleichmittel und das biologische Material umfaßt, verweilt vorzugsweise etwa 5 bis 10 min im Mischer, bevor es am Überlauf als fertiges Produkt den Mischer verläßt und anschließend konfektioniert abgefüllt wird.

Die Qualität des Produkts wird direkt nach dem Mischer (01) bestimmt, indem in regelmäßigen Abständen der Peroxidgehalt (01006) und der pH-Wert (01007) gemessen werden. Gegebenenfalls wird die Dosiermenge der Zuschlagsstoffe anschließend nachgeregelt. Die Intensität des Mischvorgangs im Mischer (01) kann durch die Drehzahleinstellung der Schaufelmischelemente variiert werden.

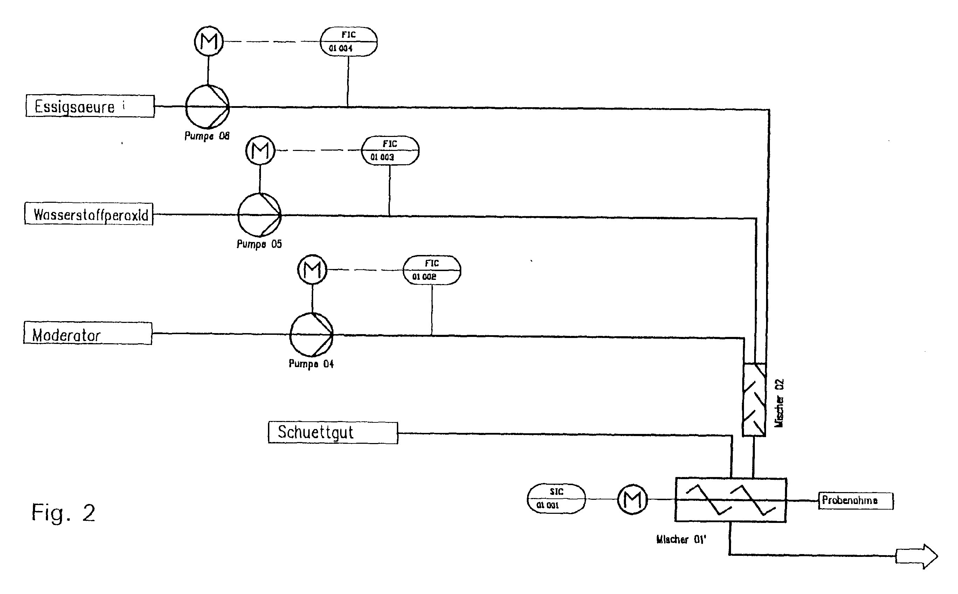

Figur 2 zeigt eine Ausführungsform der vorliegenden Erfindung mit einem chargenweisen Mischvorgang. Dabei wird eine definierte Menge (Charge) des gemahlenen biologischen Materials in den Mischer (01') eingebracht und anschließend werden die flüssigen Inhaltsstoffe mit Dosierpumpen (04) bis (06) in einem Mischer (02), beispielsweise einen Statikmischer, homogenisiert und als Bleichmittel im Mischer (01') auf das mechanisch fluidisierte Rohmaterial aufgesprüht. Nach einer Reaktionszeit von vorzugsweise 5 bis 10 min wird der Mischvorgang unterbrochen, eine Probe entnommen und der Peroxidgehalt und der pH-Wert gemessen. Entsprechend dem Probeergebnis wird gegebenenfalls erneut ein Mischvorgang definiert durchgeführt oder das fertige Produkt zur Weiterverarbeitung aus dem Mischer entleert.

Daraufhin wird das Produkt in geeignete Behälter, wie beispielsweise Container, Säcke oder BigBags, abgefüllt, in welchen die Nachreifung stattfindet.

Unmittelbar nach der Behandlung mit dem Bleichmittel weist das behandelte biologische Material einen pH-Wert von höchstens 5,0, vorzugsweise etwa 3,2 bis 4,4, besonders bevorzugt 3,2 bis 4,0, bestimmt als 10%-ige Suspension in Wasser, auf. Der Wassergehalt beträgt vorzugsweise von etwa 9 bis 15 Gew.-%, gemessen durch die Karl-Fischer-Methode. Der Peroxidgehalt beträgt vorzugsweise von etwa 250 bis 600 ppm, bestimmt durch Titration mit Cersulfatlösung.

Das erfindungsgemäße Verfahren weist somit nicht nur den Vorteil auf, daß keine Ablaugen anfallen, sondern gegenüber dem Bleichen in Suspension auch der Filtrationsschritt und der Trocknungsschritt nach dem Bleichen entfallen können. Somit ist eine einstufige Herstellung des erfindungsgemäßen Abrasivums möglich. Für einen Reiniger notwendige weitere Komponenten, wie Tenside und Konsistenzgeber können dadurch auch direkt zugegeben werden. Ferner ist das Abrasivum auf Basis von biologischem Material in einer höheren Ausbeute erhältlich.

Die vorliegende Erfindung betrifft ferner ein Abrasivum auf Basis von biologischem Material für kosmetische Präparate, welches durch das erfindungsgemäße Verfahren herstellbar ist. Das erfindungsgemäße Abrasivum basiert auf den vorstehend beschriebenen biologischen Materialien. Insbesondere betrifft sie ein Abrasivum für kosmetische Präparate auf Basis von biologischem Material, wobei das biologische Material aus Gräsern, Teilen von Stämmen oder Ästen von Gehölzen, Spelzen von Getreide, Luffa Gurken, Schwämmen und Rattan ausgewählt ist, und das Abrasivum einen Keimgehalt von höchstens 500 KBE/g und einen Gehalt an Carbonsäuren von höchstens 8 Gew.-%, vorzugsweise mindestens 0,1 Gew.-%, bezogen auf das Gewicht des Abrasivums, enthält. Vorzugsweise weist das Abrasivum einen Keimgehalt von höchsten 100 KBE/g, auf (KBE: Koloniebildende Einheiten). Die Teilchengröße des Abrasivums beträgt vorzugsweise 10 bis 2000 *µ* m, besonders bevorzugt 50 bis 200 *µ*m. Die Carbonsäuren sind entsprechend der eingesetzten Peroxysäuren vorzugsweise Essigsäure und/oder Ameisensäure.

Der Mischung können ferner ein oder mehrere Zuschläge, wie Verdicker und Pigmente, zugegeben werden.

Erfindungsgemäß brauchen dem gewünschten Material nach der Behandlung mit Aktivsauerstoff keine Konservierungsstoffe zugesetzt werden. Der geringe Anteil von Carbonsäuren, wie z.B. Essigsäure, im erfindungsgemäßen Abrasivum begünstigt als schwach saurer Puffer jedoch den Einsatz von milden bzw. gut verträglichen Konservierungsstoffen, wie beispielsweise Benzoesäure und deren Derivate, in den kosmetischen Endprodukten. Ein geringer Anteil an Carbonsäure bedeutet dabei erfindungsgemäß höchstens 8 Gew.-% Carbonsäure, vorzugsweise mindestens 0,1 Gew.-%, bezogen auf das Gewicht des Abrasivums, mehr bevorzugt 0,5 bis 6 Gew.-%. Das erfindungsgemäße Abrasivum weist vorzugsweise einen PH-Wert von höchstens 5,0, mehr bevorzugt zwischen 3,2 bis 4,4, noch mehr bevorzugt zwischen 3,2 bis 4,0 auf. Auf den Einsatz von Pigmenten und optischen Aufhellern kann weitgehend verzichtet werden. Durch die Behandlung mit Aktivsauerstoff wird die mikrobiologische Aktivität des biologischen Materials soweit reduziert, daß es auch ohne weitere Behandlung, wie Bestrahlung oder andere harte Konservierungsmittel, gelagert und in kosmetischen Produkten eingesetzt werden kann.

Dem erfindungsgemäßen Abrasivum können ferner Zuschläge zum Zerstören von überschüssigem Peroxid oder pH-Moderatoren zugesetzt werden. Beispielsweise können Laugen, wie Soda, Pottasche, Wasserglas, Kalkmilch, Milchsäure oder Reduktone, wie Ascorbinsäure und Isoascorbinsäure, eingesetzt werden.

Die vorliegende Erfindung betrifft ferner die Verwendung des erfindungsgemäßen Abrasivums auf Basis von biologischem Material für kosmetische Präparate, insbesondere Handreiniger und Peelingprodukte. Das erfindungsgemäße Abrasivum kann aber auch für milde Scheuermittel auf dem Reinigungssektor verwendet werden.

Bei einer Verwendung als Grobhandreiniger wird das erfindungsgemäße Abrasivum vorzugsweise mit Basistensiden, wie Ethersulfaten, Cotensiden, wie Sulfosuccinaten, Zuckertensiden und Betainen, Konservierungsmitteln, Konsistenzgebern, wie Salzen, Carboxymethylcellulose (CMC), Bentoniten, Xanthanen und Polyacrylaten, Lösemitteln, Duftstoffen, Aromen und Puffern bzw. Laugen oder Säuren zur pH-Stabilisierung und gegebenenfalls optischen Aufhellern gemischt.

Bei einer Verwendung in einem wasserfreien bzw. wasserarmen Reiniger, d.h. einem Reiniger mit einem Wassergehalt von 0 bis 15 Gew.-%, wird das erfindungsgemäße Abrasivum vorzugsweise mit Tensiden, wie Niotenside, Sulfosuccinate und Betaine, Konsistenzgebern, wie Salze, CMC, Bentonite, Xanthane, Polyacrylate, Wachse und SiO₂, Konservierungsmitteln, Feuchtigkeitsspendern, wie Glycerin und Propylenglycol, Duftstoffen, Aromen, Lösemitteln, wie langkettige verzweigte und unverzweigte Alkane und Ester, und Puffern bzw. Laugen oder Säuren zur pH-Stabilisierung und gegebenenfalls optischen Aufhellern gemischt.

Bei einer Verwendung in einem milden Scheuermittel wird das erfindungsgemäße Abrasivum vorzugsweise mit Tensiden, wie Cocoamidopropylbetain, Natriumisethionat und Natriumlaurylsulfat, Fließhilfsmitteln, wie modifiziertes Siliciumdioxid, Schmutzträgern, wie Carboxymethylcellulose, Hydroxyethylcellulose und Stärke, Duftstoffen und Aromen gemischt.

### Beispiele

### A. Kontinuierliche Herstellung

| Rezeptur 1: | | |
|---|---|---|
| Inhaltsstoffe | bevorzugte Bereiche [Gew.-%] | Beispiel 1 Gehalt [Gew.-%] |
| Wasserstoffperoxid 50%ig | 2-5 | 3,500 |
| Wasser | 1-5 | 1,517 |
| Essigsäure 90-100%ig | 2-5 | 3,200 |
| Schwefelsäure 98%ig | 0,001-2 | 0,083 |
| gemahlenes biologisches Material | 90-95 | 91,700 |

Wasserstoffperoxid, Wasser, Essigsäure und Schwefelsäure werden vor dem Aufsprühen auf das kontinuierlich zugeführte biologische Material in dem in der Tabelle angegebenen Verhältnis gemischt. Das Vermischen findet unmittelbar vor dem Aufsprühen mittels eines Statikmischers in den Zuleitungen des Mischers statt. Die Verweildauer der Inhaltsstoffe beträgt nach der Zugabe des Bleichmittels etwa 5 bis 10 Minuten, bevor das Material den Contimischer verläßt. Dann wird das behandelte biologische Material abgefüllt, wonach eine bis zu 24-stündige Nachreifung stattfindet. Nach etwa 10 Tagen ist im fertigen Produkt kein Peroxid mehr nachweisbar.

### B. Chargen-weise Herstellung

| Rezeptur 2: | | |
|---|---|---|
| Inhaltsstoffe | bevorzugte Bereiche [Gew.-%] | Beispiel 2 Gehalt [Gew.-%] |
| Peroxyessigsäure | 2-5 | 8,300 |
| Natronlauge, 50%ig | 1-2 | 1,000 |
| Erfindungsgemäßes Abrasivum | 90-95 | 90,700 |

Auf das vorgelegte Bambusmehl wird die mit dem Moderator vorgemischte Peroxyessigsäure über einen Zeitraum von 10 Minuten aufgesprüht und gerührt. Einen ausreichenden Füllgrad des Mischers und eine genügende Rührintensität vorausgesetzt, wird bis zu 30 Minuten nachgerührt. Anschließend wird das behandelte Bambusmehl abgefüllt, woraufhin eine bis zu 24-stündige Nachreifung stattfindet. Unmittelbar nach der Herstellung weist die Mischung die folgende Produktspezifikation auf:

| | |
|---|---|
| Peroxidgehalt (quantitativ als Titration mit Cersulfatlösung) | etwa 250-600 ppm |
| pH-Wert (10%-ig in Wasser) | etwa 3,2-4,0 |
| Wassergehalt (quantitativ nach der Karl-Fischer-Methode) | etwa 9-15 Gew.-% |

Nach etwa 10 Tagen ist im fertigen Produkt kein Peroxid mehr nachweisbar.

### C. Batch-weise Herstellung mit Zuschlägen von Pigmenten und Verdickern

| Rezeptur 3: | | |
|---|---|---|
| Inhaltsstoffe | bevorzugte Bereiche [Gew.-%] | Beispiel 3 Gehalt [Gew.-%] |
| Peroxyessigsäure | 3-10 | 8,3 |
| Natronlauge, 50%ig | 0,1-1 | 1,0 |
| Bambusmehl | 40-60 | 46,0 |
| Bentonit | 20-50 | 35,0 |
| Carboxymethylcellulose | 10-20 | 14,5 |

Auf das vorgelegte Bambusmehl wird die mit dem Moderator vorgemischte Peroxyessigsäure über einen Zeitraum von 10 Minuten aufgesprüht und gerührt. Einen ausreichenden Füllgrad des Mischers und eine genügende Rührintensität vorausgesetzt, wird bis zu 30 Minuten nachgerührt. Zur Sicherung der gewünschten Produktqualität wird eine Probe des Produkts aus dem Mischer genommen und der Peroxidgehalts und der pH-Wert gemessen. Liegen die Werte außerhalb der Produktspezifikationen, wird nochmals Bleichmittel und/oder Moderator im entsprechenden Verhältnis zugesetzt und vermischt, bis die entsprechende Produktqualität erreicht ist.

im Anschluß daran werden Carboxymethylcellulose und Bentonit zugesetzt und gründlich mit dem behandelten Bambusmehl vermischt.

Anschließend wird die resultierende Mischung beispielsweise in Großgebinde abgefüllt, in denen die Nachreifung stattfindet. Das Produkt enthält damit bereits weitere für beispielsweise einen Grobhandreiniger benötigte Zusatzstoffe im richtigen Verhältnis.

Unmittelbar nach der Herstellung, d.h. nach der Zugabe des Verdickungsmittel und des Füllstoffes, weist die Mischung die folgende Produktspezifikation auf:

| | |
|---|---|
| Peroxidgehalt (quantitativ als Titration mit Cersulfatlösung) | etwa 250-500 ppm |
| pH-Wert (10%-ig in Wasser) | etwa 3,2-4,4 |
| Wassergehalt (quantitativ nach der Karl-Fischer-Methode) | etwa 9-15 Gew.-% |

Nach etwa 10 Tagen ist im fertigen Produkt kein Peroxid mehr nachweisbar.

### D. Beispielrezeptur für einen Grobhandreiniger

| Rezeptur 4: | | |
|---|---|---|
| | bevorzugter Bereich [Gew.-%] | Beispiel 4 [Gew.-%] |
| Wasser | 30-70 | 50 |
| erfindungsgemäßes Abrasivum | 2-30 | 15 |
| Natriumlaurylsulfat | 5-20 | 15 |
| Decylglucosid | 1-10 | 5 |
| Bentonit/Xanthanlösung | 0,1-5 | 5 |
| Dinatrium-PEG-5-laurylcitrat-sulfosuccinat | 1-10 | 5,4 |
| Titandioxid | 0,05-2 | 0,5 |
| Citronensäure | 0,1-2 | 0,5 |
| Duftstoff(e) | 0,05-1 | 0,5 |
| 2-Brom-2-nitropropan-1,3-diol | 0,1 | 0,1 |

### E. Beispielrezeptur für einen wasserfreien bzw. wasserarmen Reiniger

| Rezeptur 5: | |
|---|---|
| | Beispiel 6: Gehalt [Gew.-%] |
| Bienenwachs | 3 |
| Mineralöl | 10 |
| Steareth-2 | 7 |
| Steareth-10 | 7 |
| Laureth-3 | 10 |
| Dimethylgluratat, -adipat, -succinat | 40 |
| Silica 27/98 | 2 |
| Milchsäure/Natriumlactat | 1,5 |
| Erfindungsgemäßes Abrasivum | 15 |
| Glycerin | 4,5 |

### F. Beispielrezeptur für ein Peeling

| Rezeptur 6: | |
|---|---|
| | Beispiel 7: Gehalt [Gew.-%] |
| Erfindungsgemäßes Abrasivum | 15,0 |
| PEG-8 | 10,0 |
| Stearinsäure | 8,97 |
| Glycerin | 5,10 |
| Paraffinum Liquidum | 2,50 |
| Cetylpalmitat | 1,50 |
| Ammoniumstearat | 1,09 |
| Cellulose Gum | 1,00 |
| Magnesiumsulfat | 0,27 |
| Methylparaben | 0,11 |
| Propylparaben | 0,11 |
| Duftstoff(e) | 0,10 |
| Tocopherol | 0,02 |
| Wasser | ad 100 |

### G. Beispielsrezeptur für ein Handreinigerkonzentrat

| Rezeptur 7: | |
|---|---|
| | Beispiel 8: Gehalt [Gew.-%] |
| erfindungsgemäßes Abrasivum | 31,9 |
| Natriumlaurylsulfat | 31,9 |
| Decylglucosid | 10,6 |
| Bentonit/Xanthanlösung | 10,6 |
| Dinatrium-PEG-5-laurylcitrat-sulfosuccinat | 11,5 |
| Titandioxid | 1,1 |
| Citronensäure | 1,1 |
| Duftstoff(e) | 1,1 |
| Konservierungsmittel | 0,2 |

### H. Beispielsrezeptur für ein Scheuermittelkonzentrat

| Rezeptur 8: | | |
|---|---|---|
| | bevorzugter Bereich [Gew.-%] | Beispiel 9 Gehalt [Gew.-%] |
| Natriumisethionat | 10-20 | 15 |
| Cocoamidopropylbetain | 2-7 | 5 |
| Natriumlaurylsulfat | 2-7 | 5 |
| erfindungsgemäßes Abrasivum | 50-80 | 68 |
| Maisstärke | 2-7 | 5 |
| modifiziertes Siliciumdioxid | 1-4 | 2 |

## Patentansprüche

1. Verfahren zur Herstellung eines Abrasivums für kosmetische Präparate, umfassend das Mahlen eines biologischen Materials und das Mischen des mindestens einen gemahlenen biologischen Materials mit einem Bleichmittel, wobei die das biologische Material und das Bleichmittel umfassende Mischung höchstens 60 Gew.-% Wasser enthält und das unmittelbar nach der Behandlung mit dem Bleichmittel erhaltene biologische Material einen pH-Wert von höchstens 5,0, bestimmt als 10%-ige Suspension in Wasser, aufweist.

2. Verfahren nach Anspruch 1, wobei das biologische Material aus Gräsern, Teilen von Stämmen oder Ästen von Gehölzen, Spelzen von Getreide, Luffa Gurken, Rattan oder einer Mischung von einem oder mehreren von diesen ausgewählt ist.

3. Verfahren nach Anspruch 1 oder 2, wobei ein Bleichmittel verwendet wird, welches mindestens eine organische Peroxidverbindung umfaßt.

4. Verfahren nach Anspruch 3, wobei die organische Peroxidverbindung Peroxyameisensäure oder Peroxyessigsäure ist.

5. Verfahren nach einem der vorhergehenden Ansprüche, wobei das biologische Material auf eine Korngröße von 10 bis 2000 *µ*m gemahlen wird.

6. Verfahren nach einem der vorhergehenden Ansprüche, wobei das Bleichmittel in einem Anteil von 1 bis 40 Gew.-%, bezogen auf das Gesamtgewicht der das Bleichmittel und das biologische Material umfassenden Mischung, verwendet wird.

7. Verfahren nach einem der vorhergehenden Ansprüche, wobei ein Bleichmittel verwendet wird, welches ferner mindestens einen Moderator in einem Anteil von bis zu 5 Gew.-%, bezogen auf das Gesamtgewicht der das Bleichmittel und das biologische Material umfassenden Mischung, enthält.

8. Verfahren nach einem der vorhergehenden Ansprüche, wobei ein Bleichmittel mit einem Wassergehalt von bis zu 10 Gew.-%, bezogen auf das Gesamtgewicht der das Bleichmittel und das biologische Material umfassenden Mischung, verwendet wird.

9. Abrasivum für kosmetische Präparate auf Basis von biologischem Material, wobei das biologische Material aus Gräsern, Teilen von Stämmen oder Ästen von Gehölzen, Spelzen von Getreide, Luffa Gurken, Schwämmen und Rattan ausgewählt ist und das Abrasivum einen Keimgehalt von höchstens 500 KBE/g und einen Gehalt an Carbonsäuren von höchstens 8 Gew.-%, bezogen auf das Gewicht des Abrasivums, enthält.

10. Abrasivum nach Anspruch 9, wobei das biologische Material eine Teilchengröße von 10 bis 2000 *µ*m aufweist.

11. Verwendung eines Abrasivums nach Anspruch 9 oder 10 in einer Formulierung als Hand- oder Hautreiniger.
